# EUROPEAN PATENT APPLICATION

(11) **EP 2 910 209 A1**
(43) Date of publication of application: **26.08.2015**
(21) Application number: 14156097.9
(22) Date of filing: 21.02.2014
(51) Int. Cl.: A61B 17/72

(54) **Intramedullary device for osteosynthesis**

(71) Applicant: NG Patentverwertungs UG, 40223 Düsseldorf (DE)
(72) Inventor: Lahme, Steffen, 34513 Waldeck-Sachsenhausen (DE)
(74) Representative: Remus, Alvaro Johannes

(57) **Abstract**

The invention relates to a device 1, in particular for insertion into the medullary cavity of a fractured tubular bone, comprising at least one outer element 2 which comprises at least one expandable section 3, 4, at least one inner element 12 which is at least partially disposed within the outer element 2 and at least one operating means 13 which is operable to force the expandable section 3, 4 from an unexpanded state to an expanded state. The operating means 13 is coupled with the expandable section 3, 4 such that the operating means 13 is further operable to force the expandable section 3, 4 from the expanded state to the unexpanded state. Thus, the operating means 13 is operatively coupled to the inner element 12 for reversibly expanding the expandable sections 3, 4. To this end, nut-like tip piece 6 acts as a coupling means that interacts with the inner element 12 so as to provide an operative connection between the inner element 12 and the expandable sections 3, 4.

## Description

### Background of the invention

The invention relates to a device, in particular for insertion into the medullary cavity of a fractured tubular bone, comprising at least one outer element which comprises at least one expandable section, at least one inner element which is at least partially disposed within the outer element and at least one operating means which is operable to force the expandable section from an unexpanded state to an expanded state. For example, the invention relates to an intramedullary osteosynthesis nail for repositioning and stabilization of fractured long bones.

### Prior art

Rod-like devices that can be introduced into the medullary cavity of a hollow tubular bone are commonly referred to as intramedullary nails (IM nails) and well known to treat fractures of long bones of the human or animal body. IM nails result in earlier return to activity for the patients since they share the load with the bone, rather than entirely supporting the bone. Usually, IM nails have a rotationally stable clover-leaf shape. However, several modifications and shapes are used for various bones such as V-nails for tibia, radius and ulna. IM nails are usually made of metal, in particular titanium, which has several advantages, including higher mechanical stability and improved biocompatibility. In order to prevent collapse or rotation in inherently unstable fractures, IM nails are often fixed using bolts on each end of the nail. That is, the nail is fixed to the bony cortex so that rotation among the bone fragments is prevented.

In the context of uniting fractured bone portions or bridging sound bone portions with replacement parts, a particular problem is that of bone fractures which require the use of intramedullary nails which are received in the marrow canal and secured in place by transverse pins. Various types of intramedullary nails are already known, which are expandable in a limited section of the nail in order to allow the fixation of the nail against the bone cortex, e. g. by means of radially deploying a number of blades in the distal portion of the intramedullary nail.

For example, DD 293 485 A5 discloses an intramedullary nail including a load bearing feed rod and a plurality of cylindrical hollow elements which are slidably mounted on the feed rod. Some of the hollow elements are radially expandable while the other hollow elements are rigid spacers disposed between two radially expandable elements. The hollow element that is disposed at the distal end of the feed rod comprises an internal thread which serves as an anchor for an external thread of the feed rod. Radial expansion of the respective hollow elements within a fractured bone is achieved by tightening the feed rod so as to compress the hollow elements and thereby causing radial expansion of the elements so that the intramedullary nail is fixed in the medullary cavity of the bone. However, it is a disadvantage of this known IM nail that the radial expansion of the hollow elements is not reversible so that removal or replacement of the nail is impossible or at least difficult.

WO 97/18769 A1 discloses an intramedullary nail including an axial rod supporting tubular spacers and expandable, releasable and interchangeable sleeves slidably mounted on the axial rod between an axial abutment and a control nut. Tightening the control nut axially compresses the spacers and the expandable sleeves, and thereby causes radial expansion of the expandable sleeves in order to lock the intramedullary nail in the marrow cavity of a bone. The expandable sleeves comprise a substantially continuous, tubular, flexible and expandable circumferential wall made of a biocompatible material and operative to prevent bone rehabilitation inside the sleeves. But also with this IM nail, it is a drawback that the expansion of the expandable sleeves is not reversible so that removal of this known nail is impossible or at least difficult either.

EP 1 227 765 B1 discloses an intramedullary nail for fixation of bone fractures with a head, a tip and a longitudinal axis, whereby a first radially expandable section is provided in the proximal section adjacent to the head of the intramedullary nail. A second radially expandable section is provided in the distal section adjacent to the tip of the intramedullary nail, and a non-expandable middle section is provided between said two radially expandable sections of the intramedullary nail. By compressing the radially expandable sections the IM nail can be fixed in the medullary cavity of the bone as the expanded sections are pressed against the inner surface of the medullary cavity. However, removal of this known nail is also at least difficult since the expansion of the expandable sections is not reversible either.

Accordingly, it is a common drawback of the prior art IM nails that nail removal, renewal or replacement is sophisticated or almost impossible. Further disadvantages of conventional IM nails are localized stresses and a lack of opportunity for accurate positioning within the bone cavity.

### Summary of the invention

It is the object of the present invention to provide a device for insertion into the medullary cavity of a fractured tubular bone, which can be easily removed, renewed or replaced and accurately positioned within the bone cavity, and with which localized stresses within the bone cavity are reduced.

The object is met by providing a device as initially specified, wherein the operating means is coupled with the expandable section such that the operating means is further operable to force the expandable section from the expanded state to the unexpanded state. That is, the operating means of the device is operatively coupled with the expandable section so as to enable adjustment of the state of the expandable section in both directions, i.e. from an unexpanded state to an expanded state and *vice versa*. Accordingly, the expansion of the expandable section is reversible so that the device according to the invention can be easily removed, renewed or replaced. Moreover, the state of the expandable sections can be accurately adjusted by actuating the operating means as it is easily possible to adjust the expandable sections to any position between the completely expanded and the completely compressed (unexpanded) state. Such reversible alteration of the state of the expandable section allows for precise adjustment and accurate positioning of the device within the bone cavity. Optimization of the contact pressure by means of appropriate adjustment further results in minimization of local stresses at the inner surface of the bone cavity. Thus, undesired damage or even blasting of the treated bone can be avoided. The device according to the invention is therefore a very useful and effective tool for treating fractures of long bones of the human or animal body.

In one embodiment of the invention the operating means can be operatively coupled to the inner element for reversibly expanding the expandable section. The device according to the invention may further comprise at least one coupling means that interacts with the inner element so as to provide an operative connection between the inner element and the expandable section. Due to these features of the device according to the invention, operative coupling between the operating means and the expandable section is provided so that the expandable section can be expanded or compressed in vertical direction by using the operating means, wherein the inner element and the coupling means act as intermediate elements that transmit the actuation of the operating means to the expandable section.

The operating means may be disposed outside the outer element while the inner element may be completely or at least substantially disposed within the outer element. The inner element can be a compact element that bears almost the entire load, i.e. mainly shares the load with the damaged bone, while the outer element only contributes less to the stabilization of the bone.

The coupling of the operating means to the expandable section may be realized, for example, by means of a bolted connection comprising a screw-like inner element which includes an external thread and a nut-like coupling means which includes an internal thread. In such embodiment, the nut-like coupling means of the device according to the invention may be part of the outer element and the screw-like inner element may be partially disposed within the coupling means.

For example, the inner element may comprise an external thread at its distal end and the coupling means may comprise an internal thread within the outer element that corresponds to the external thread of the inner element. In an alternative embodiment, the inner element may comprise an internal thread at its distal end and the coupling means may comprise an external thread within the outer element that corresponds to the internal thread of the inner element.

The outer element of the device according to the invention may comprise, for example, at least two expandable sections and at least one intermediate section disposed between the expandable sections. It is a particular advantage of this embodiment that the whole device is shortened when the expandable sections are expanded. The reduction of the length of the device leads to a contraction of the site of fracture if the device is inserted into the medullary cavity such that the site of fracture lies between the two expandable sections. That is, if the device is fixed within the medullary cavity, a possible gap between two parts of a fractured bone can be closed due to the length reduction of the device according to the invention. In this embodiment the expandable sections may each be connected with the intermediate section which can be, for example, a rigid tubular element.

In one embodiment of the invention the expandable section comprises a tapered radius or tip in the expanded state in order to tightly fix the device within the medullary cavity and thus prevent collapse or rotation in inherently unstable fractures.

In order to further enhance the rotation-resistance of the device within the medullary cavity, it is provided that the expandable section comprises a V-shaped or arrow-like cutout. If the expandable section is compressed in longitudinal direction and thus expanded vertically, this cutout is unfolded and produces a kind of arrowhead or spike protruding the expanded section and penetrating the inner surface of the bone. Accordingly, the V-shaped or arrow-like cutout results in additional stabilization of the device, in particular in respect of radial forces, and enhanced power intake in rotation. Alternatively, the expandable section may comprise a V-shaped or arrow-like attachment which is unfolded when the expandable section is expanded and then forms a kind of arrowhead or spike which protrudes the respective expanded section. This attachment can be a kind of plate or sheet which is attached to the outer surface of the expandable section.

In another embodiment of the invention the expandable section is at least partially covered by a sleeve. The sleeve may be, for example, made of silicone or any other biocompatible material and protects the bone against undesired damage by the expanded section.

The outer element may have a decreasable length and the inner element may be shorter than the outer element, even in a state of maximally decreased length of the outer element. That is, the inner element does not protrude the outer element at the distal end of the device according to the invention in any state.

In one embodiment of the invention the two expandable sections of the outer element are disposed such that the distance between them is maximized. Due to this measure, the device according to the invention can be fixed within the bone with maximum stability.

In a further embodiment of the invention the outer element and the inner element are at least partially bended with respect to their longitudinal axis so that the device according to the invention can be used with bended bones as well.

The expandable section of the device according to the invention may comprise at least two bendable bars which can be separated and/or spaced from each other.

According to an exemplary embodiment of the invention the outer element and the inner element each extend along a longitudinal axis and the expandable section is expandable approximately perpendicular to the longitudinal axis, i.e. in vertical direction.

In one embodiment of the invention at least one of the inner element, the coupling means and the operating means comprises a bore extending along the longitudinal axis and entire length of at least one of the inner element, the coupling means and the operating means. For example, a wire might be introduced into this bore in order to guide the device according to the invention during its introduction into the medullary cavity, in particular if a complicated fracture has split the bone into pieces.

The object is further met by providing a device for insertion into the medullary cavity of a fractured tubular bone, comprising at least one outer element which comprises at least one expandable section, at least one inner element which is at least partially disposed within the outer element and at least one operating means which is operable to force the expandable section from an unexpanded state to an expanded state, wherein the outer element has a decreasable length and the inner element is shorter than the outer element, even in a state of maximally decreased length of the outer element. Accordingly, the inner element does not protrude the outer element at the distal end of the device according to the invention in any state.

The device according to the invention may be made, e.g., of titanium or any biocompatible, stable and light metal or suitable polymer. The dimensions of the device, e.g., length and diameter, can be adapted to the kind of bone to be treated and the specific application.

The invention is further exemplarily described in detail with reference to the drawings.

### Brief description of the drawings

**Figure 1** shows a longitudinal section of an exemplary embodiment of a device according to the invention in the compressed state.
**Figure 2** shows a longitudinal section of the device according to figure 1 in the expanded state.
**Figure 3** shows a longitudinal section of the tip piece of the device according to figure 1.
**Figure 4** shows a longitudinal section of the inner jacket of the device according to figure 1.
**Figure 5** shows a longitudinal section of the inner element of the device according to figure 1.
**Figure 6** shows a longitudinal section of the head piece of the device according to figure 1.
**Figure 7** shows a schematic plan view of the device according to figure 1.
**Figure 8** shows a schematic perspective view of a bendable bar of the device according to figure 7.

### Detailed description of exemplary and preferred embodiments of the invention

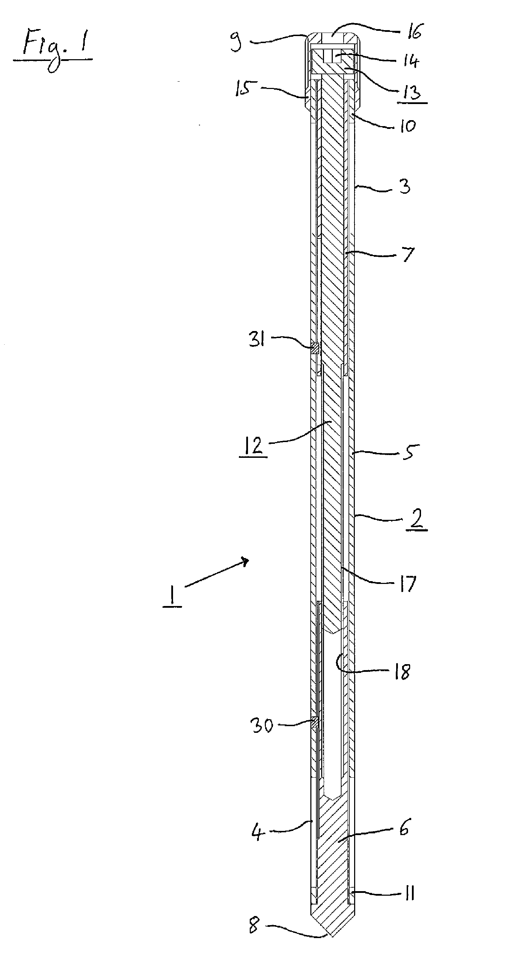

**Figure 1** shows a longitudinal section of an exemplary embodiment of a device 1 according to the invention. The device 1 comprises an outer element 2 comprising two expandable sections 3, 4 which are connected to each other via an intermediate section 5. The intermediate section 5 can be, for example, a rigid tubular element. In this embodiment, the intermediate section 5 is permanently attached to the expandable sections 3, 4, i.e. integrally fixed to them. That is, the expandable sections 3, 4 and the intermediate section 5 are integral parts of the outer element 2, wherein these sections can be formed, for example, as one piece. The outer element 2 further comprises a tip piece 6 and a jacket 7, wherein the tip piece 6 is disposed at the distal end 8 of the device 1 while the jacket 7 is disposed at the proximal end 9 of the device 1. The proximal expandable section 3 is firmly attached to the jacket 7 via a proximal end section 10 and the distal expandable section 4 is firmly attached to the tip piece 6 via a distal end section 11. For example, the end sections 10, 11 can be fixed to the jacket 7 and the tip piece 6, respectively, by welding. The expandable sections 3, 4 may be attached to the respective end sections 10, 11 in the same way as they each are attached to the intermediate section 5. The intermediate section 5 is further fixed to the tip piece 6 and the jacket 7 in the rotational sense by means of the slot nuts 26, 27, respectively.

The device 1 further comprises an inner element 12 which is disposed within the outer element 2. In this embodiment, the inner element 12 is a screw or bolt which is permanently attached to an operating means 13 comprising an adaptor 14, for example, for a corresponding tool or instrument which is designed to actuate the operating means 13. As becomes apparent from figure 1, the operating means 13 is disposed outside the outer element 2 while the inner element 12 is completely or at least substantially disposed within the outer element 2. The operating means 13 is covered by a head piece 15 which is attached to the end section 10 of the outer element 2. The head piece 15 has an opening 16 at the distal end 9, through which a tool or instrument for actuating the operating means 13 can access the adaptor 14. The inner element 12 is operatively coupled to the operating means 13 for reversibly expanding the expandable sections 3, 4 and hence acts as a kind of adjusting means for adjusting the state of the expandable sections 3, 4. To this end, at its distal end the inner element 12 engages with the tip piece 6 which acts as a coupling means so that the inner element 12 is operatively coupled to the expandable sections 3, 4. This coupling of the operating means 13 to the expandable sections 3, 4 is accomplished in this example by means of a bolted connection comprising the screw-like inner element 12 which includes an external thread 17 and the nut-like tip piece 6 which includes an internal thread 18. In this exemplary embodiment, the coupling means (nut-like tip piece 6) of the device 1 is part of the outer element 2 and the screw-like inner element 12 is at least partially disposed within this coupling means.

In a particular embodiment of the invention (not shown here) the operating means 13, the inner element 12 and the coupling means (nut-like tip piece 6) each comprise a bore extending along the longitudinal axis and entire length of the operating means, the inner element and the coupling means. For example, a wire might be introduced into this bore in order to guide the device according to the invention during its introduction into the medullary cavity, in particular if a complicated fracture has split the bone into pieces.

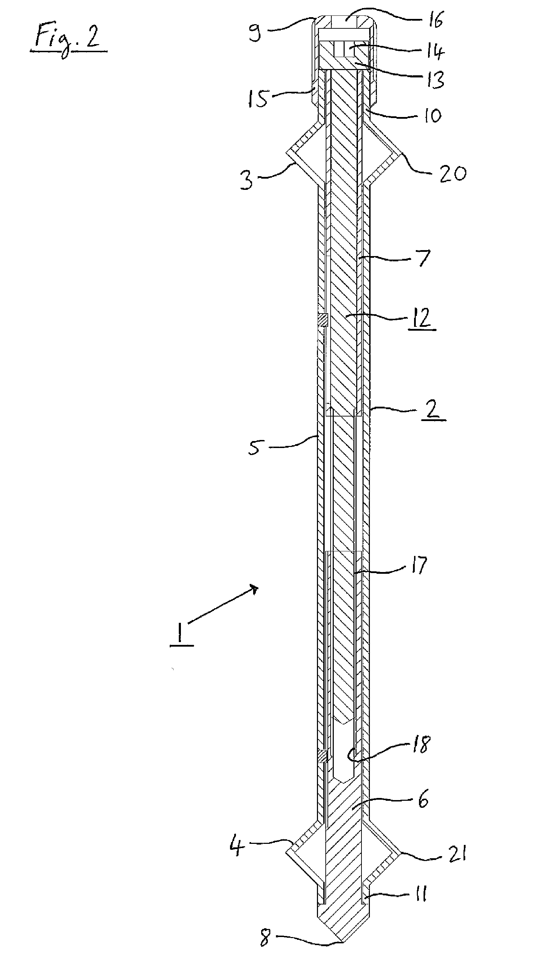

In figure 1 the expandable sections 3, 4 of the device 1 are compressed, i.e. not expanded in vertical direction. If the operating means 13 is actuated by an appropriate tool or instrument, i.e. by turning the operating means 13 in clockwise or anti-clockwise direction (depending on the orientation of the threads of the bolted connection), the state of the expandable sections 3, 4 can be adjusted. Figure 2 shows the device 1 with expandable sections 3, 4 being expanded in vertical direction.

**Figure 2** shows a longitudinal section of the device 1 according to figure 1 in the expanded state. As explained above, the operating means 13 is operatively coupled to the inner element 12 for reversibly expanding the expandable sections 3, 4. To this end, nut-like tip piece 6 acts as a coupling means that interacts with the inner element 12 so as to provide an operative connection between the inner element 12 and the expandable sections 3, 4. Thus, operative coupling between the operating means 13 and the expandable sections 3, 4 is provided so that the expandable sections 3, 4 can be expanded or compressed (unexpanded) in vertical direction by actuating the operating means 13. Accordingly, the inner element 12 and the nut-like tip piece 6 act as intermediate elements that transmit the actuation of the operating means 13 to the expandable sections 3, 4. If the operating means 13 is actuated by an appropriate tool or instrument, i.e. by turning the operating means 13 in a first direction (clockwise or anti-clockwise direction, depending on the orientation of the threads of the bolted connection), the inner element 12 pulls the tip piece 6 towards the proximal end 9 and hence exerts force on the distal end section 11 which rests on a rim 19 of the tip piece 6. As the end sections 10, 11 and the intermediate section 5 are rigid tubular elements, this force compresses the expandable sections 3, 4 in longitudinal direction and hence results in expansion of the expandable sections 3, 4 in vertical direction as depicted in figure 2.

If the operating means 13 is actuated by the tool or instrument, i.e. by turning the operating means 13 in a second direction opposite to the first direction (clockwise or anti-clockwise direction, depending on the orientation of the threads of the bolted connection), the inner element 12 pushes the tip piece 6 towards the distal end 8 and hence exerts a tensile force on the distal end section 11 which is rigidly mounted to the tip piece 6, e.g., by welding. As the end sections 10, 11, the expandable sections 3, 4 and the intermediate section 5 are all rigidly connected to each other and thus linked up, this tensile force stretches the expandable sections 3, 4 in longitudinal direction and hence results in compression of the expandable sections 3, 4 in vertical direction. That is, the expansion of the expandable sections 3, 4 is reversible so that the expanded state of the device 1 as depicted in figure 2 can be easily transferred into the unexpanded state of the device 1 as depicted in figure 1. Moreover, the state of the expandable sections 3, 4 can be accurately adjusted by actuating the operating means 13, i.e. turning it both directions, as it is easily possible to adjust the expandable sections 3, 4 to any position between the completely expanded and the completely unexpanded state. This kind of beneficial fine tuning allows for minimization of local stresses at the inner surface of the bone cavity so as to avoid undesired damage or even blasting of the treated bone.

As becomes apparent from figure 2, the expandable sections 3, 4 each comprise a tapered radius or tip 20, 21 in the expanded state. These tapered tips 20, 21 ensure tight fixation of the device 1 within the medullary cavity and thus prevent collapse or rotation in inherently unstable fractures.

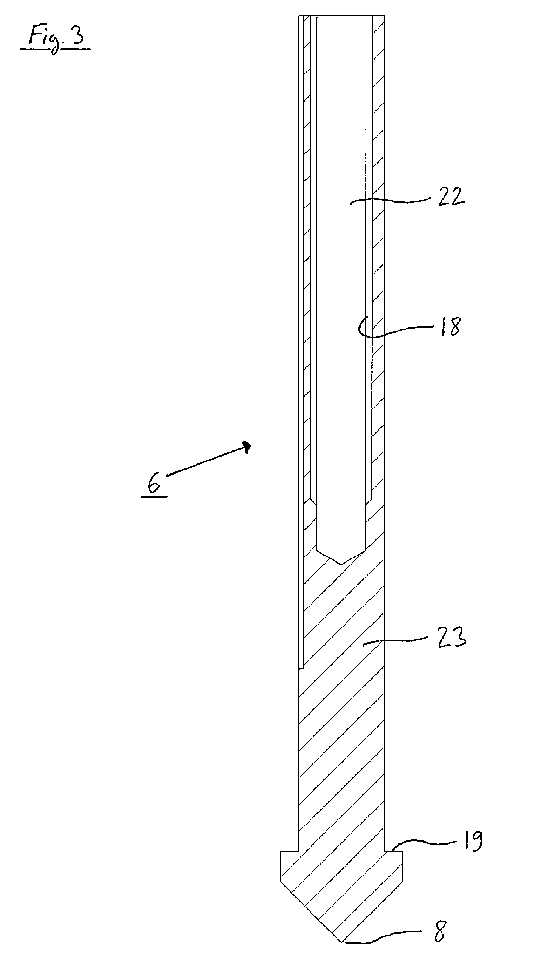

**Figure 3** shows an enlarged view of the tip piece 6 of the device 1. As becomes apparent, the tip piece 6 is a tubular element comprising a one-end bore 22 for receiving the inner element 12 (not depicted in this figure) and a compact section 23. The bore 22 comprises the internal thread 18 which corresponds to the external thread 17 of the inner element 12. The rim 19 serves as a support for the distal end section 11 of the outer element 2 (not depicted in this figure).

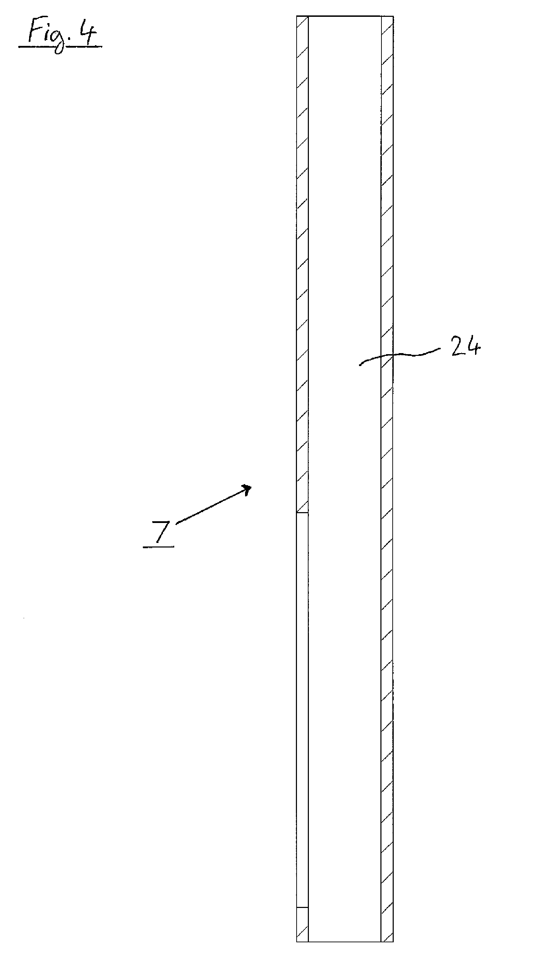

**Figure 4** shows an enlarged view of the jacket 7 of the device 1. The jacket 7 is a hollow tubular element which comprises an end-to-end bore 24 for receiving the inner element 12 (not depicted in this figure). As the tip piece 6 shown in figure 3 has a wider radius than the inner element 12, the jacket 7 is needed to stabilize the inner element 12 within the outer element 2 (not depicted in this figure).

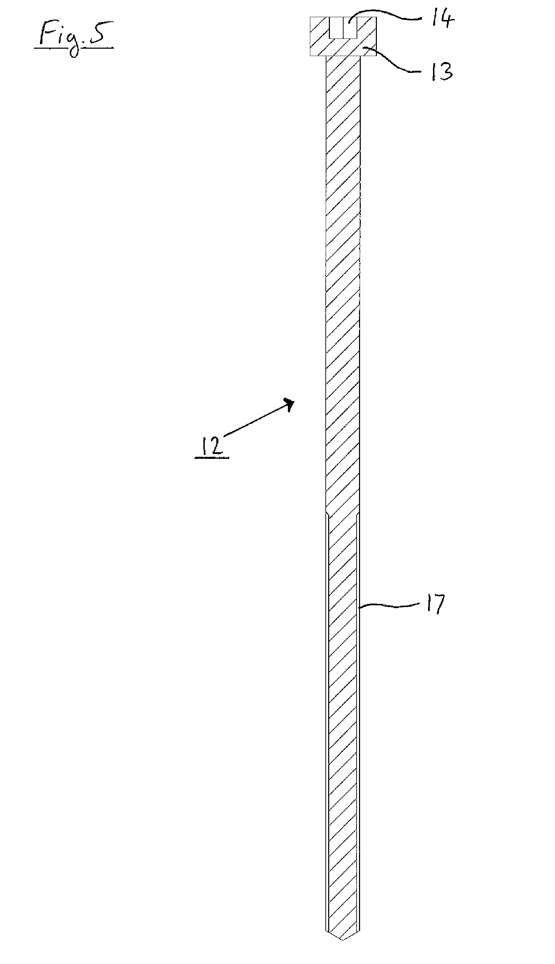

**Figure 5** shows an enlarged view of the inner element 12 of the device 1. The inner element 12 is a compact screw- or bolt-like element including an external thread 17 which corresponds to the internal thread 18 of the tip piece 6 shown in figure 3. In this exemplary embodiment the inner element 12 is rigidly and permanently attached to the operating means 13 which comprises the adaptor 14 for receiving a corresponding tool or instrument, e.g., a socket wrench, designed to actuate the operating means 13. By means of the tool or instrument the operating means 13 can be turned in clockwise or anti-clockwise direction. The compact inner element 12 is the component of the device 1 according to the invention that bears almost the entire load, i.e. mainly shares the load with the damaged bone. The other components, in particular the outer element, only contribute less to the stabilization of the bone.

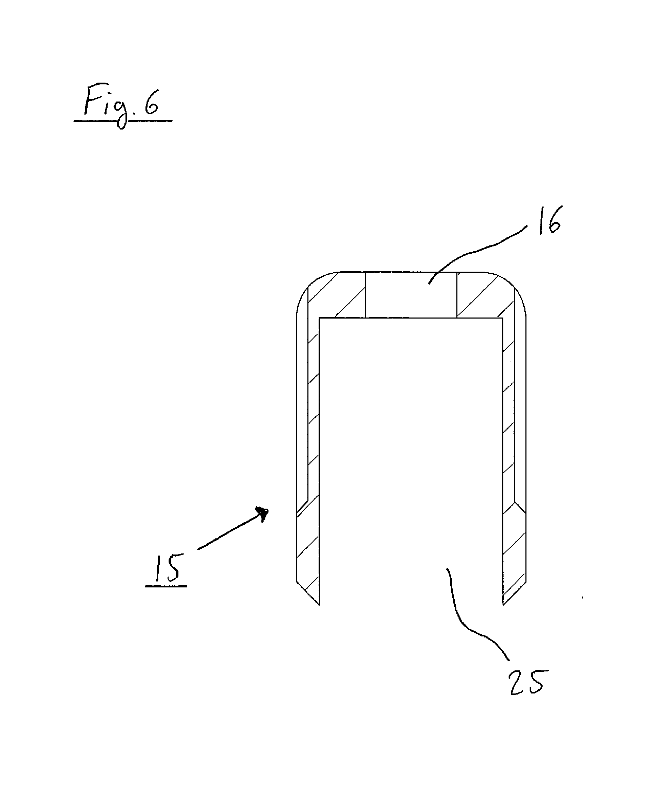

**Figure 6** shows an enlarged view of the head piece 15 of the device. The head piece 15 has an opening 16 through which a tool or instrument can access the operating means 13 (not depicted in this figure). The head piece 15 can be used as a cover for the operating means 13 and may be attached to the outer element 2 (not depicted in this figure). To this end, the head piece 15 comprises a recess 25 which is designed to receive the proximal end 9 of the device 1. The head piece 15 protects the operating means 13 and reduces the risk of undesired invasion of material from the bone cavity into the device 1.

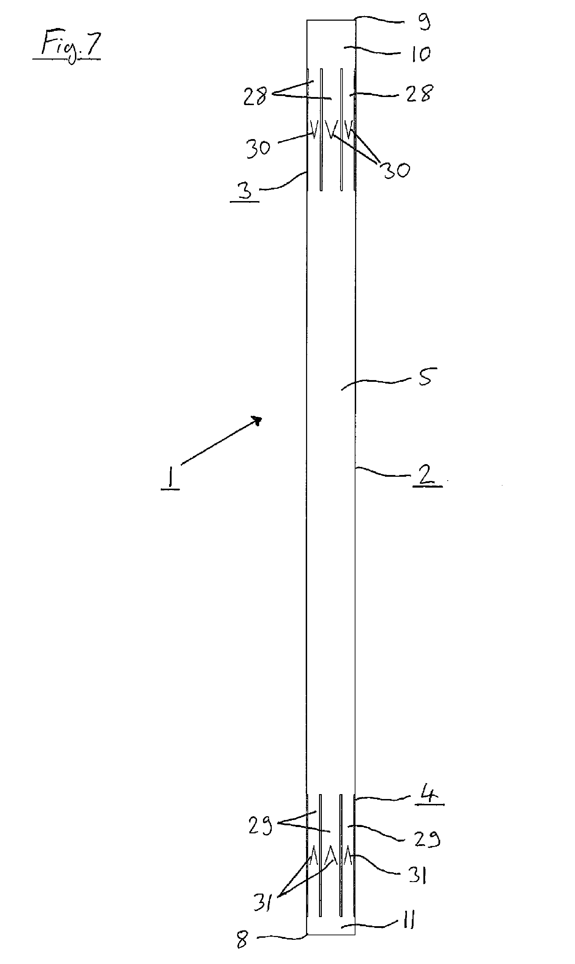

**Figure 7** shows a schematic plan view of the expandable sections 3, 4 of the device 1. The expandable sections 3, 4 each comprise bendable bars 28, 29 which are distributed around the circumference of the outer element 2 and which are spaced from each other. In this embodiment, the intermediate section 5 and the proximal end section 10 are each permanently attached to the expandable section 3, i.e. integrally fixed to it. Similarly, the intermediate section 5 and the distal end section 11 are each permanently attached to the expandable section 4. That is, the expandable sections 3, 4, the end sections 10, 11 and the intermediate section 5 are integral parts of the outer element 2, wherein in this example these sections 3, 4, 5, 10, 11 are formed as one piece. The bars 28, 29 of the expandable sections 3, 4 each comprise a V-shaped or arrow-like cutout 30, 31. If the expandable sections 3, 4 are compressed in longitudinal direction and thus expanded vertically, these cutouts 30, 31 are unfolded and each produce a kind of arrowhead or spike protruding the expanded sections 3, 4. Thus, the V-shaped or arrow-like cutouts 30, 31 result in additional stabilization of the device 1, in particular in respect of radial forces, and hence enhanced rotation-resistance. It is a further advantage of this embodiment that the tips of the cutouts 30 at the proximal end 9 as well as the tips of the cutouts 31 at the distal end 8 are each directed towards the intermediate section 5, so that they form arrowheads or spikes facing each other and penetrating the inner surface of the bone like a pair of nippers if the device 1 is shortened when the expandable sections 3, 4 are expanded as described above. Alternatively, at least one expandable section 3, 4 may comprise a V-shaped or arrow-like attachment which is unfolded when the respective bar 28, 29 is expanded and then forms a kind of arrowhead or spike which protrudes the respective expanded bar 28, 29. This attachment can be a kind of plate or sheet which is attached to the outer surface of the respective bar.

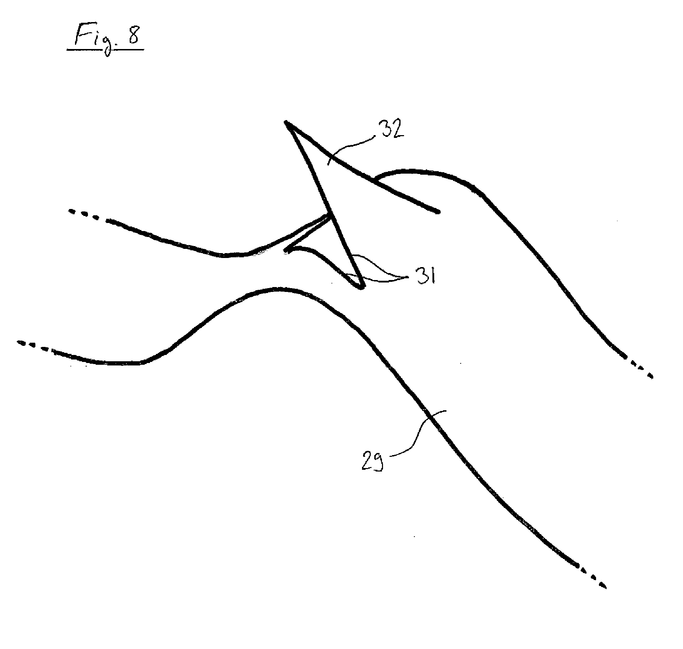

**Figure 8** shows a schematic representation of a bendable bar 29 of the distal expandable section 4 of the device 1 according to figure 7. The expandable section 4 comprises an expandable bar 29 which is provided with a V-shaped or arrow-like cutout 31. If the expandable section 4 is in an expanded state (as depicted in this figure), the bar 29 is expanded in vertical direction. In this state, the cutout 31 is unfolded and forms a kind of arrowhead or spike 32 which protrudes the expanded bar 29.

### List of reference numbers

- 1: Device
- 2: Outer element
- 3: Expandable section
- 4: Expandable section
- 5: Intermediate section
- 6: Tip piece
- 7: Jacket
- 8: Distal end
- 9: Proximal end
- 10: End section
- 11: End section
- 12: Inner element
- 13: Operating means
- 14: Adaptor
- 15: Head piece
- 16: Opening
- 17: Thread
- 18: Thread
- 19: Rim
- 20: Tip
- 21: Tip
- 22: Bore
- 23: Compact section
- 24: Bore
- 25: Recess
- 26: Slot nut
- 27: Slot nut
- 28: Bars
- 29: Bars
- 30: Cutouts
- 31: Cutouts
- 32: Spike

## Claims

1. Device (1), in particular for insertion into the medullary cavity of a fractured tubular bone, comprising at least one outer element (2) which comprises at least one expandable section (3, 4), at least one inner element (12) which is at least partially disposed within the outer element (2) and at least one operating means (13) which is operable to force the expandable section (3, 4) from an unexpanded state to an expanded state, **characterized in that** the operating means (13) is coupled with the expandable section (3, 4) such that the operating means (13) is further operable to force the expandable section (3, 4) from the expanded state to the unexpanded state.

2. Device according to claim 1, **characterized in that** the operating means (13) is operatively coupled to the inner element (12) for reversibly expanding the expandable section (3, 4).

3. Device according to claim 1 or 2, further comprising at least one coupling means that interacts with the inner element (12).

4. Device according to any one of claims 1 to 3, **characterized in that** the outer element (2) comprises at least two expandable sections (3, 4) and at least one intermediate section (5) disposed between the expandable sections (3, 4).

5. Device according to claim 4, **characterized in that** the expandable sections (3, 4) each are connected with the intermediate section (5).

6. Device according to claim 4 or 5, **characterized in that** the intermediate section (5) is a rigid tubular element.

7. Device according to any one of claims 1 to 6, **characterized in that** the expandable section (3, 4) comprises a tapered radius or tip (20, 21) in the expanded state.

8. Device according to any one of the claims 1 to 7, **characterized in that** the expandable section (3, 4) comprises a V-shaped or arrow-like cutout (30, 31) or attachment.

9. Device according to any one of claims 1 to 8, **characterized in that** the expandable section (3, 4) is at least partially covered by a sleeve.

10. Device according to any one of claims 1 to 9, **characterized in that** the outer element (2) has a decreasable length and the inner element (12) is shorter than the outer element (2), even in a state of maximally decreased length of the outer element (2).

11. Device according to any one of claims 4 to 10, **characterized in that** the two expandable sections (3, 4) of the outer element (2) are disposed such that the distance between them is maximized.

12. Device according to any one of claims 1 to 11, **characterized in that** the outer element (2) and the inner element (12) are at least partially bended with respect to their longitudinal axis.

13. Device according to any one of claims 1 to 12, **characterized in that** the expandable section (3, 4) comprises at least two bendable bars (28, 29) which are separated and/or spaced from each other.

14. Device according to any one of claims 1 to 13, **characterized in that** the outer element (2) and the inner element (12) each extend along a longitudinal axis and the expandable section (3, 4) is expandable approximately perpendicular to the longitudinal axis.

15. Device according to any one of claims 1 to 14, **characterized in that** at least one of the inner element (12), the coupling means and the operating means (13) comprises a bore extending along the longitudinal axis and entire length of at least one of the inner element (12), the coupling means and the operating means (13).
